(19) Europäisches Patentamt
European Patent Office
Office européen des brevets

(11) Numéro de publication: **0 045 338**
**B1**

(12) # FASCICULE DE BREVET EUROPÉEN

(45) Date de publication du fascicule du brevet: **05.11.86**

(51) Int. Cl.⁴: **A 61 K 31/715**

(21) Numéro de dépôt: **80401154.2**

(22) Date de dépôt: **05.08.80**

(54) **Utilisation des glycosylglucanes en gastroentérologie dans le traitement des colopathies.**

(43) Date de publication de la demande:
**10.02.82 Bulletin 82/6**

(45) Mention de la délivrance du brevet:
**05.11.86 Bulletin 86/45**

(84) Etats contractants désignés:
**BE CH DE FR GB IT LI LU NL SE**

(56) Documents cité:
**FR-A-2 406 447**
**US-A-3 301 848**
**US-A-3 436 311**
**US-A-3 987 166**

**Industrial Guns", 2d Ed. (1973), p. 488-511**
**"Kirk Othmer Encyclopedia of Chemical Technology", 3d Ed./5, p. 454,455**

(73) Titulaire: **LABORATOIRES DEBAT Société anonyme dite:, 60 rue de Monceau, F-75008 Paris (FR)**

(72) Inventeur: **Nguyen Cong Duc, Amaury, Château du Boisson Brécy, F-02210 Oulchy le Chateau (FR)**

(74) Mandataire: **Combe, André, CABINET BEAU DE LOMENIE 55 rue d'Amsterdam, F-75008 Paris (FR)**

**EP 0 045 338 B1**

Il est rappelé que: Dans un délai dé neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

On sait du brevet américain n° 3 415 929 que l'on a déjà préconisé l'utilisation de poly-1,3-β-glucosides à chaîne polysaccharidique linéaire, dans des compositions ophtalmologiques, en tant que véhicule pour prolonger l'action de principes actifs oculaires administrés par voie locale.

On sait des brevets américains n° 3 507 290 et 3 659 025 que l'on a préconisé l'utilisation de polysaccharides ramifiés, ayant une chaîne linéaire poly-1,3-glucopyranosides ramifiée latéralement par des motifs 1,6-glucopyranoses, en cosmétique soit pour la mise en plis des cheveux, soit en association avec des émollients et de l'eau pour les soins de la peau.

On vient de trouver de façon surprenante que certains polysaccharides des brevets américains n° 3 507 290 et 3 659 025, appartenant à la famille des glycosylglucanes de formule I ci-après, sont utiles en thérapeutique par voie orale, notamment en gastroentérologie et en particulier en tant qu'agents améliorant le transit intestinal pour le traitement des colopathies.

La présente invention concerne donc l'utilisation d'un glycosylglucane répondant à la formule générale :

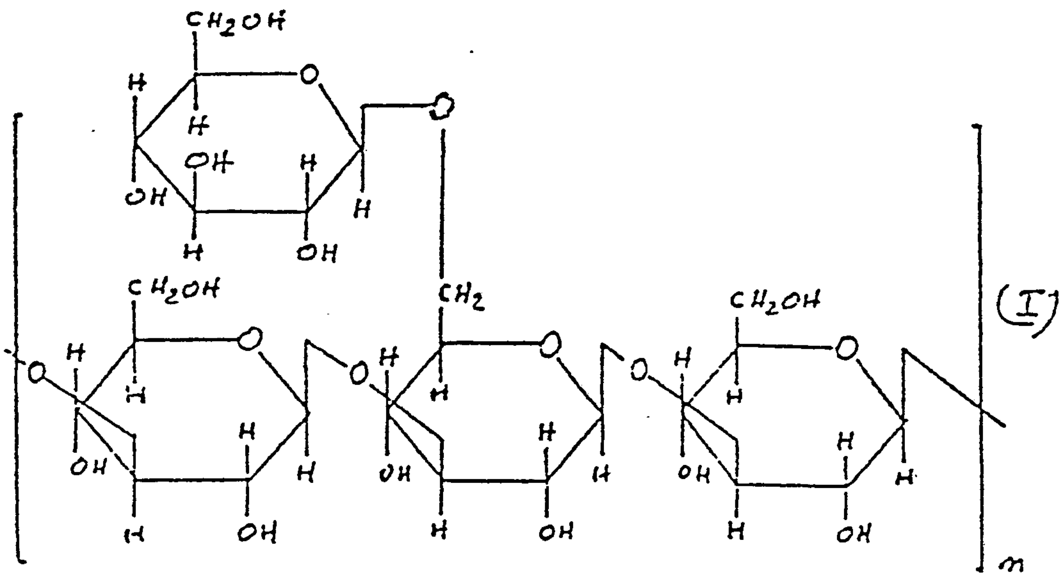

(I)

(où n est un nombre entier) et dont le poids moléculaire est supérieur ou égal à 50.000, pour la fabrication d'un médicament pour le traitement de colopathies, par voie orale.

Les glycosylglucanes sont donc des polymères présentant comme motif unitaire une chaîne de trois groupes 1,3 glucopyransyle, le groupe central présentant comme substituant un groupe 1,6-glucopyransyle. La formule développée I peut être représentée schématiquement par la formule condensée:

$$\left[\begin{array}{ccccc} & & (1,6)-G & & \\ & & | & & \\ -(1,3)-G & — & (1,3)-G & — & (1,3)-G- \end{array}\right]_n$$

(où n est défini comme ci-dessus et G représente un reste glucopyranosyle $C_6H_{10}O_5$).

On utilise selon l'invention des polymères ayant un poids moléculaire (PM) moyen compris entre 130.000 et 1.200.000, c'est-à-dire des produits dont le degré de polymérisation n est approximativement compris entre environ 200 et environ 1.850.

Parmi ces polymères, les plus intéressants, tant sur le plan des propriétés gastroentérologiques que celui de la préparation industrielle, notamment par fermentation, sont ceux qui ont un PM compris entre 500.000 et 600.000, parmi ces derniers le glycosylglucane préféré selon l'invention est celui qui a un degré de polymérisation n de l'ordre de 800 environ.

Les glycosylglucanes de formule I sont des homopolysaccharides non ioniques qui présentent les propriétés physico-chimiques caractéristiques suivantes:

A - VISCOSITE:

La viscosité des sols augmente en fonction de la concentration (cf. fig. 1). Pour une concentration donnée, elle augmente en fonction du temps d'hydratation pour arriver à une valeur maximale en 12-24 h environ (cf. fig. 2). Elle est pratiquement indépendante du pH et est stable, toutes choses étant égales d'ailleurs, entre pH 0

et pH 12,5. Elle n'est pas influencée par les variations de températures entre 15° C et 90° C.

B - INSENSIBILITE AUX ELECTROLYTES:

Les glycosylglucanes sont compatibles, dans une gamme très large de pH (pH O à pH 12,5), avec la plupart des acides, bases et sels. Aucun sel, même à ion chromique, n'a d'effet de réticulation prononcé.

C - POUVOIR GONFLANT:

Les glycosylglucanes de formule I présentent un bon pouvoir gonflant vis-à-vis de l'eau et des liquides corporels (sérum, plasma).

D - RHEOLOGIE:

Les sols ont des propriétés rhéologiques pseudoplastiques plus marquées que celles des autres colloïdes connus notamment dans le domaine gastroentérologique. La pseudoplasticité se traduit par un seuil d'écoulement élevé en-dessous duquel le fluide reste immobile, et par une diminution instantanée de la viscosité dès qu'il y a mouvement ou agitation. Ce phénomène est réversible (cf. fig. 3). Il résulte de ces propriétés rhéologiques (forte viscosité au repos, fluidité accrue en fonction du mouvement) que les glycosylglucanes selon l'invention sont autolubrifiants.

Le produit préféré (polymère de formule I ayant un degré de polymérisation n de l'ordre de 800) qui a pour No de code DU-80, possède les propriétés spécifiques suivantes:

1°) - La variation de son pouvoir rotatoire α en milieu alcalin (NaOH 0,13N à 0,01N; c'est-à-dire à un pH de 13,1 à 12) a été étudiée (sur appareil FICA SPECTROPOL 1 B) à 25°C, sous azote, à la concentration de 0,72 g/1 dans l'eau. Dans ces conditions on a α

$$\alpha_D^{25} = 100°/ml/g$$

2°) - La variation de sa viscosité (exprimée en mPas) en fonction de la concentration dans l'eau (exprimée en % en poids/volume) a été déterminée sous dispersant, à 20° C (appareil BROOKFIELD LVF, aiguille n° 3, 30 tours/mn) et est représentée par le faisceau 1 de la fig. 1.

3°) - La variation de sa viscosité (exprimée en mPas) en fonction du temps d'hydratation a été déterminée sans dispersant, à 20° C et à la concentration de 1% en poids/volume dans de l'eau (appareil BROOKFIELD LVF, aiguille n° 3, 30 tours/minute) et est représentée par la courbe de la fig. 2 (par commodité, cette courbe est tracée avec un changement d'échelle en abscisse: temps en minutes jusqu'à 60 mn, puis temps en heures au-delà de 1 h). On observe que en 12-24 h la viscosité du DU-80, à la concentration de 1 % (poids/volume) dans de l'eau, atteint la valeur maximale de 4 Pas 4000 cPo).

4°) - Pour apprécier sa pseudoplasticité, on a étudié la variation de la viscosité (exprimée en cPo) en fonction de la vitesse de cisaillement au moyen d'un viscosimètre BROOKFIELD LVF (aiguille n° 3, à 6, 12, 30 et 60 tours/mn). La courbe de la fig. 3 (qui a été enregistrée sur un cylindre) montre l'évolution de la viscosité d'un sol de DU-80 (concentration de l'ordre de 1,4 % en poids/volume dans de l'eau) que l'on agite à partir du repos (V = 0) avec une vitesse de cisaillement croissante (0 < VI < V2 < V3) et que l'on agite plus dès que la vitesse V3 a été obtenue.

5°) - Son pouvoir absorbant est très important. Le DU-80 peut absorber jusqu'à 60 fois son poids d'eau sans s'écouler.

Les glycosylglucanes de formule I peuvent être préparés selon une méthode connue en soi. Ils peuvent être obtenus par fermentation selon le procédé décrit dans les brevets américains US-A-3 507 290 et US-A- 3 659 025 précités.

Ils sont utilisés en gastroentérologie. Ils améliorent le transit intestinal et sont très intéressants dans le traitement des colopathies (atonie intestinale, paresse intestinale, constipation).

A côté de leur propriété d'améliorer le transit intestinal, ils présentent d'autres effets avantageux en thérapeutique liés à leurs propriétés d'agents gonflants: ils peuvent en particulier agir en tant qu'agents anorexigènes (en donnant une sensation de satiété quand ils tapissent la paroi de l'estomac) et en tant qu'agents sequestrant les acides biliaires.

Selon l'invention, on préconise l'utilasation d'une composition thérapeutique utile en gastroentérologie dans le traitement des colopathies, caractérisée en ce qu'elle renferme, en association avec un excipient physiologiquement acceptable, au moins un glycosylglucane de formule I ayant un poids moléculaire supérieur ou égal à 50.000, le poids moléculaire étant de façon avantageuse compris entre environ 130.000 et environ 1.200.000, et de préférence compris entre 500.000 et 600.000, le glycosylglucane le plus intéressant ayant un degré de polymérisation n de l'ordre de 800.

Les glycosylglucanes préparés pour l'administration orale peuvent être conditionnés sous forme de gélules, de sachets ou de gels. L'administration chez l'homme adulte sera avantageusement effectuée sous formes de gelules pouvant comporter, si nécessaire, un agent d'écoulement ou de lubrification, tel que le stéarate de magnésium, pour renforcer les propriétés autolubrifiantes du glycosylglucane. L'administration chez l'enfant sera avantageusement effectuée sous forme de gels comportant, le cas échéant, des agents d'aromatisation et de conservation.

On a résumé ci-après les résultats des essais toxicologiques et pharmacologiques qui ont été entrepris avec

les glycosylglucanes selon l'invention.

1°) - TOXICITE:

La DL-0 (dose maximale non toxique) per os chez le rat mâle est supèrieure à 5 g/kg.

Par ailleurs les essais de toxicité chronique, per os, chez le chien et chez le rat, ont mis en évidence que les glycosylglucanes selon l'invention, à la dose quotidienne de 2 g/kg pendant 90 jours, n'entrainent aucune mortalité.

2°) - TOLERANCE:

Les essais de non-irritabilité pour les yeux et les essais de tolérance dermique et intestinale ont démontré que les glycosylglucanes selon l'invention ne sont nullement agressifs vis-à-vis des muqueuses.

3°) - ACTION SUR LE TRANSIT INTESTINAL:

L'action sur le transit intestinal a été étudiée selon la technique de LOEWE et FAURE, Arch. Exp. Path. pharm. 107, 271 (1925). Des rats femelles Sprague-Dawley d'un moids moyen de 110 g répartis par lots de 10 animaux chacun (un lot par dose et par produit à tester et un lot témoin ne recevant que de l'eau), sont mis à jeun 24 h avant l'administration per os des produits dans de l'eau (sous un volume de 2 ml pour 100 g de poids corporel), on administre 30 minutes après l'administration des produits à tester une suspension de charbon (10 g de charbon végétal dans 100 ml d' eau). 20 minutes après l'administration du charbon les animaux sont sacrifiés et saignés. On prélève l'intestin grèle en le sectionnant au niveau du pylore et du caecum. On mesure la longueur totale (L) de l'intestin grèle et la longueur (1) traversée par le charbon. On détermine le rapport

$$\frac{1 \times 100}{L},$$

qui exprime en pourcentage la longueur traversée par le charbon, et l'amélioration du transit intestinal en pourcentage par rapport au lot témoin.

Les résultats obtenus, qui ont été consignés dans le tableau I, montrent que le DU-80 est significativement plus actif que le produit de référence, la gomme sterculia.

**TABLEAU I**

| Produit | Dose mg/kg | L (cm) | 1 (cm) | $\frac{1 \times 100}{L}$ | Amélioration du transit par rapport au lot témoin. |
|---|---|---|---|---|---|
| Témoin | — | 103,5 | 53,5 | 51,7 | Base 100 |
| Sterculia | 100 | 104 | 65 | 63 | 122 % |
| Sterculia | 1000 | 102 | 64 | 62 | 120 % |
| DU-80 | 12,5 | 105 | 60 | 58 | 112 % |
| DU-80 | 25 | 104 | 64 | 62 | 120 % |
| DU-80 | 50 | 106 | 62 | 59 | 114 % |
| DU-80 | 75 | 105 | 63 | 60 | 116 % |
| DU-80 | 100 | 104,5 | 70,5 | 67,5 | 131 % |
| DU-80 | 1000 | 105 | 74 | 71 | 138 % |

4°) - RESISTANCE VIS-A-VIS DU SUC PANCREATIQUE: Les essais in vitro effectués avec de la pancréatine de porc [qui comprend plusieurs enzymes (amylasa, trypsine et autres protéases, lipases et ribonucléasa)] ont

permis de vérifier que les glycosylglucanes de formule I et en particulier le DU-80, ne sont pas détruits par l'amylase da ls pancréatina de porc. En effet après hydrolyse de 22 h la quantité da glucose libre s'est avérée nulle.

## Revendications

1. Utilisation d'un glycosylglucane répondant à la formule générale :.

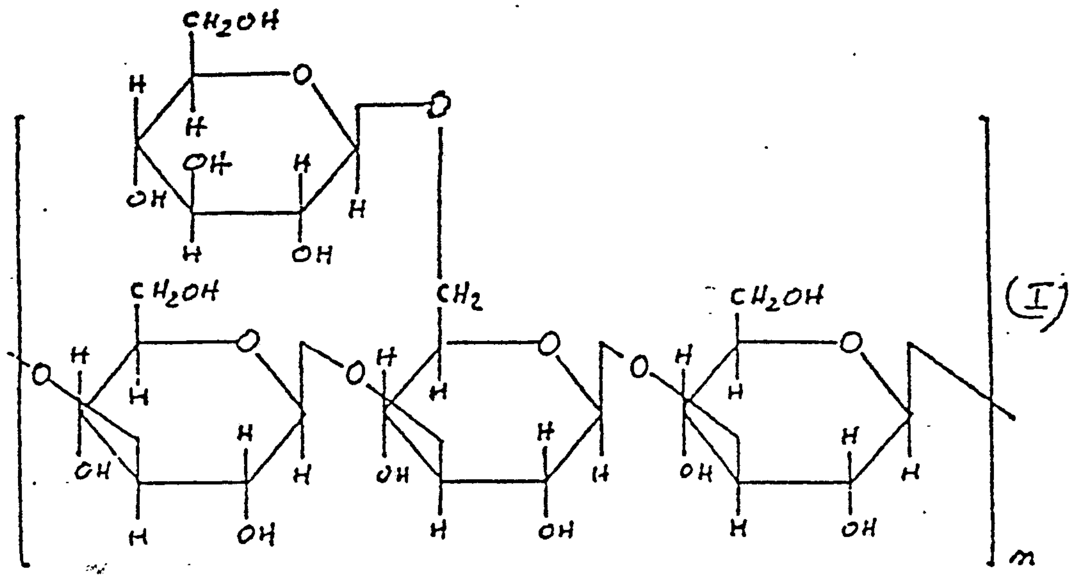

(I)

(où n est un nombre entier) et dont le poids moléculaire est supérieure ou égal à 50.000, pour la fabrication d'un médicament pour le traitement de colopathies par voie orale.

2. Utilisation selon la revendication 1 dans laquelle le glycosylglucane de formule I a un n compris entre 200 et 1850 c'est-à-dire un poids moléculaire compris entre 130.000 et 1.200.000.

3. Utilisation selon les revendications 1 et 2 caractérisée en ce que le glycosylglucane est sous forme de gel.

**Patentansprüche**

Verwendung eines Glycosylglucans der allgemeinen Formel:

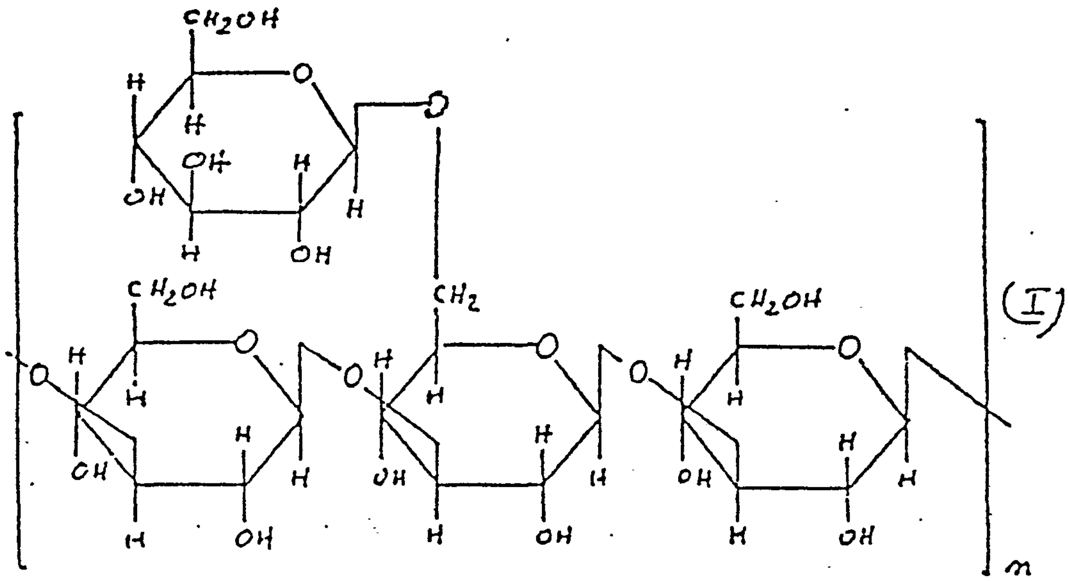

(I)

in der n eine ganze Zahl ist,
mit einem Molekulargewicht von 50 000 oder darüber zur Herstellung eines Arzneimittels zur oralen Behandlung von Erkrankungen des Dickdarms.

2. Verwendung nach Anspruch 1, dadurch gekennzeichnet, daß das Glycosylglucan der Formel I ein Molekulargewicht von 130 000 bis 1 200 000 hat, d.h.,daß n in Formel 1 einen Wert von 200 bis 1850 aufweist.

3. Verwendung nach Anspruch 1 oder 2, dadurch gekennzeichnet, daß das Glycosylglucan in Form eines Gels vorliegt.

**Claims**

1. Use of a glycosylglucan corresponding to the general formule:

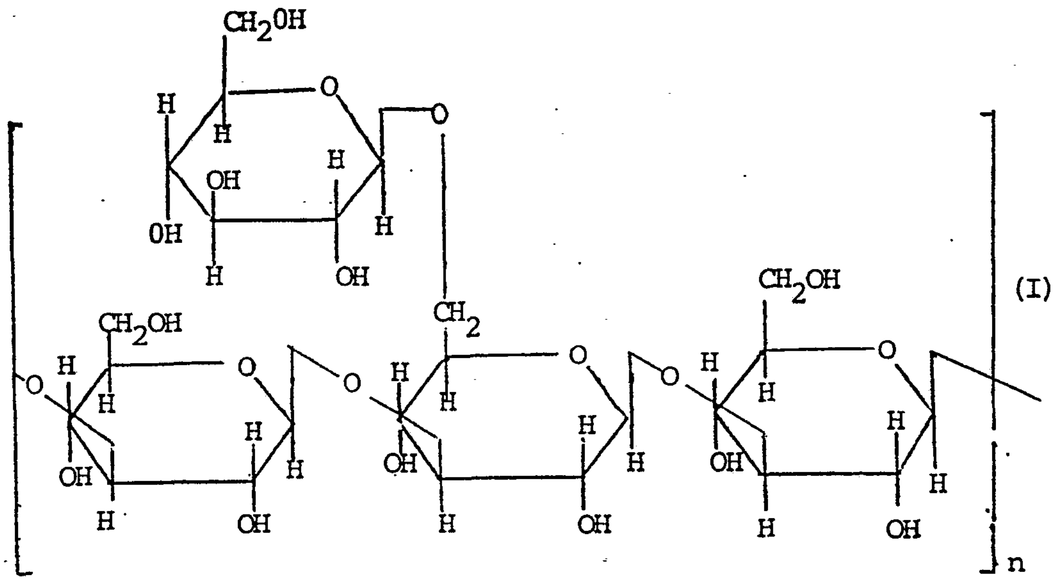

(I)

(wherein n is an integer) and the molecular weight of which is greater than of equal to 50,000, for producing a medicament for the treatment, by the oral route, of disorders of the colon.

2. Use according to claim 1 in which the glycosylgludan of formula I has a n from about 200 to about 1850, namely a molecular weight from about 130,000 to 1,200,000.

3. Use according to claims 1 and 2, characterized in that the glycosylglucan is in the form of gel.

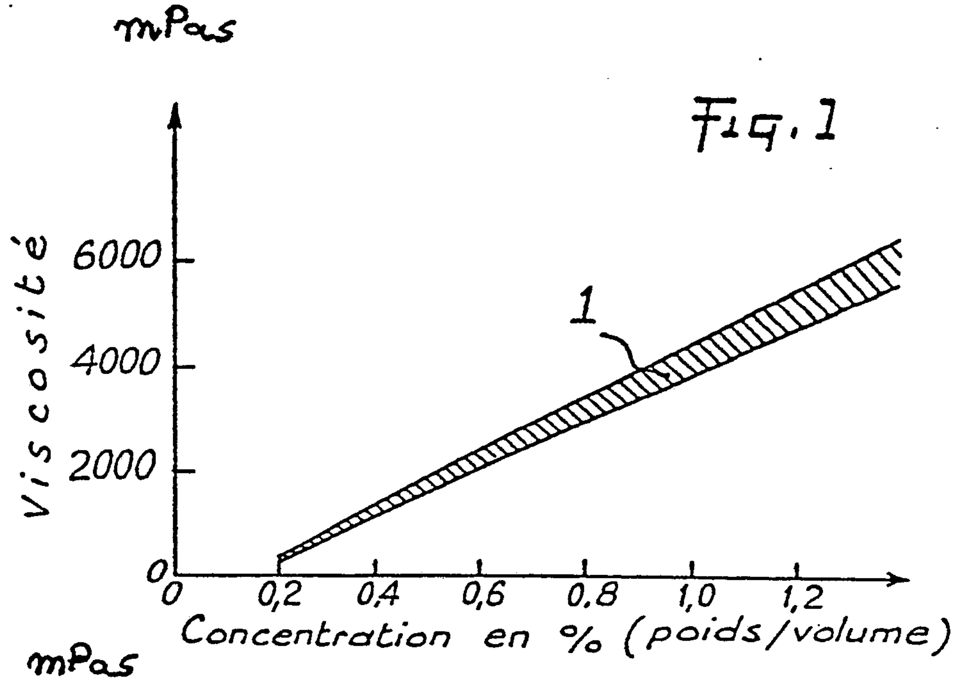


Fig. 1

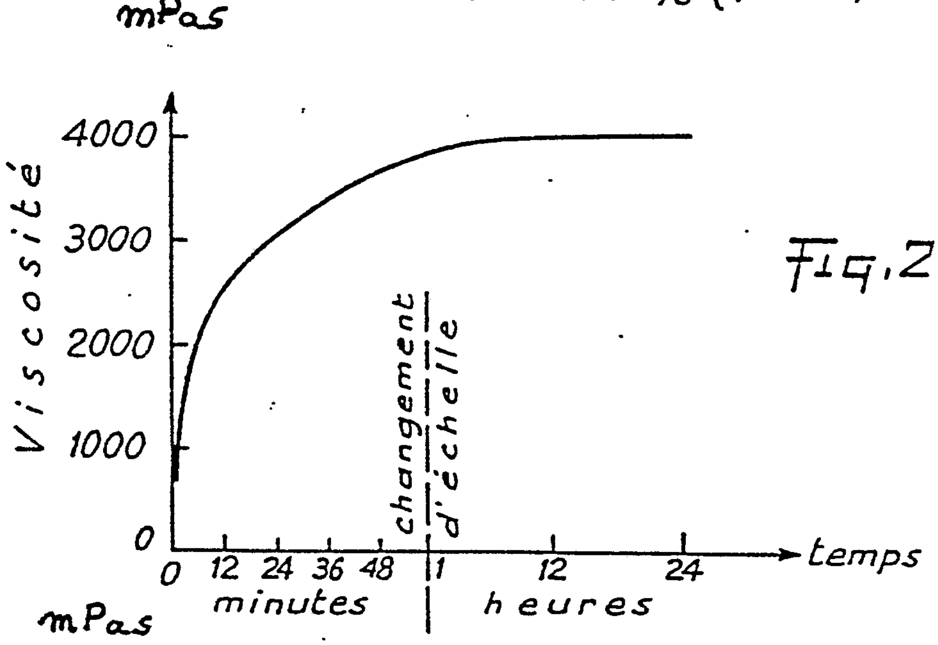


Fig. 2

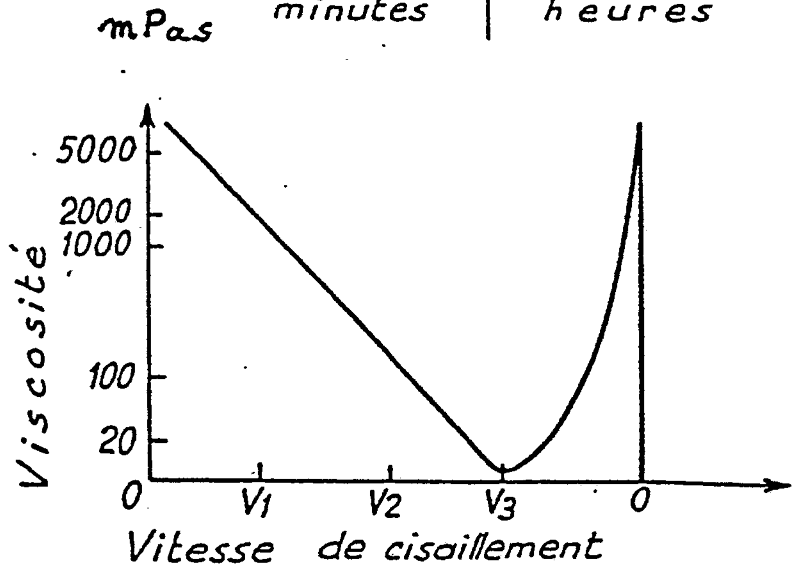


Fig. 3